# FASCICULE DE BREVET EUROPEEN

(11) **EP 3 131 847 B1**
(45) Date de publication et mention de la délivrance du brevet: **01.01.2020**
(21) Numéro de dépôt: 15725713.0
(22) Date de dépôt: 01.04.2015
(51) Int. Cl.: C07C 303/40, C01B 21/086, H01M 10/0568, H01M 10/52

(54) **PREPARATION D'IMIDES CONTENANT UN GROUPEMENT FLUOROSULFONYLE**
HERSTELLUNG VON IMIDEN MIT EINER FLUORSULFONYLGRUPPE
PREPARATION OF IMIDES CONTAINING A FLUOROSULPHONYL GROUP

(30) Priorité: 18.04.2014 FR 1453523
(43) Date de publication de la demande: 22.02.2017
(73) Titulaire: Arkema France, 92700 Colombes (FR)
(72) Inventeur: SCHMIDT, Grégory, 69700 Saint Andeol le Chateau (FR); AUDUREAU, Sophie, 69320 Feyzin (FR)
(74) Mandataire: Chahine, Audrey Claire
(86) Numéro de dépôt international: PCT/FR2015/050845
(87) Numéro de publication internationale: WO 2015/158979

(56) Documents cités:
- EP-A2- 1 122 240
- WO-A1-2009/123328
- WO-A1-2010/010613
- WO-A2-2012/160280
- US-A1- 2012 014 859
- US-A1- 2012 020 867
- US-A1- 2012 041 233
- US-A1- 2013 068 991
- MARTIN BERAN ET AL: "A New Method of the Preparation of Imido-bis(sulfuric acid) Dihalogenide, (F, Cl), and the Potassium Salt of Imido-bis(sulfuric acid) Difluoride", ZEITSCHRIFT FUER ANORGANISCHE UND ALLGEMEINE CHEMIE, vol. 631, no. 1, 1 janvier 2005 (2005-01-01), pages 55-59, XP055014688, ISSN: 0044-2313, DOI: 10.1002/zaac.200400325
- BERAN M ET AL: "A new route to the syntheses of N-(fluorosulfuryl)sulfonamide salts: Crystal structure of Ph4P(+) [CF3SO2NSO2F](-)", POLYHEDRON, PERGAMON PRESS, OXFORD, GB, vol. 29, no. 3, 19 février 2010 (2010-02-19), pages 991-994, XP026883701, ISSN: 0277-5387, DOI: 10.1016/J.POLY.2009.11.024 [extrait le 2010-01-30]

## Description

### DOMAINE DE L'INVENTION

La présente invention concerne un procédé de préparation d'imides contenant un groupement fluorosulfonyle.

### ARRIERE-PLAN TECHNIQUE

Les anions de type sulfonylimide, de par leur très faible basicité, sont de plus en plus utilisés dans le domaine du stockage d'énergie sous forme de sels inorganiques dans les batteries, ou de sels organiques dans les super condensateurs ou dans le domaine des liquides ioniques. Le marché des batteries étant en plein essor et la réduction des coûts de fabrication des batteries devenant un enjeu majeur, un procédé de synthèse à grande échelle et à bas coût de ce type d'anions est nécessaire.

Dans le domaine spécifique des batteries Li-ion, le sel actuellement le plus utilisé est le LiPF₆ mais ce sel montre de nombreux désavantages tels qu'une stabilité thermique limitée, une sensibilité à l'hydrolyse et donc une plus faible sécurité de la batterie. Récemment de nouveaux sels possédant le groupement FSO₂- ont été étudiés et ont démontré de nombreux avantages comme une meilleure conductivité ionique et une résistance à l'hydrolyse. L'un de ces sels, le LiFSI (LiN(FSO₂)₂) a montré des propriétés très intéressantes qui font de lui un bon candidat pour remplacer le LiPF₆.

Peu de procédés de synthèse du LiFSI ou de son acide correspondant ont été décrits, mais il apparait clairement que dans tous ces procédés l'étape clé est l'étape de formation de la liaison S-F.

Une première voie de synthèse décrite (Appel & Eisenbauer, Chem Ber. 95, 246-8, 1962) consiste en la réaction de l'acide fluorosulfonique (FSO₃H) avec de l'urée. Toutefois, le caractère corrosif et toxique de ce composé ne permet pas une industrialisation du procédé.

Le document EP 2415709 décrit un procédé basé sur cette voie, dans lequel les produits de la réaction de l'acide fluorosulfonique avec l'urée sont dissous dans l'eau et le bis(fluorosulfonyl)imide est précipité sous forme de sel avec du tétrabutylammonium. Cette voie de synthèse n'est pas viable à grande échelle car le rendement global est très faible.

Une autre voie consiste à faire réagir du difluorosulfoxyde avec de l'ammoniaque: voir le document WO 2010/113835 à cet égard. Mais cette méthode forme également de nombreux produits secondaires, ce qui nécessite des étapes de purification coûteuses.

Une autre voie (Ruff & Lustig, Inorg. Synth. 1968, 11, 138-43) consiste à synthétiser dans un premier temps un composé dichloré de formule (CISO₂)₂NH puis à réaliser un échange chlore/fluor avec de l'AsF₃. Toutefois, ce procédé n'est pas industrialisable du fait du prix élevé et de la toxicité de l'AsF₃.

Le document WO 02/053494 décrit une autre voie qui consiste en un échange CI/F sur le (CISO₂)₂NH à l'aide d'un fluorure de cation monovalent qui peut être alcalin ou de type onium (NR₄⁺), dans un solvant aprotique. Toutefois, d'après ce document, la réaction est très lente.

L'exemple 10 du document WO 2007/068822 décrit la synthèse du bis(fluorosulfonyl)imide dans de l'acide fluorhydrique (HF) anhydre. Ainsi, la réaction est mise en œuvre dans un autoclave avec 1 g du bis(chlorosulfonyl)imide et 4 g de HF anhydre à différentes températures et durées de réaction. Le document enseigne que même à des températures de 130°C, le rendement de la réaction ne dépasse pas 55%. En outre, il enseigne que la présence d'impuretés rend la séparation difficile à l'échelle industrielle. Il est conclu que la synthèse du bis(fluorosulfonyl)imide par l'HF n'est pas satisfaisante, et donc que l'emploi d'un fluorure de lithium est préféré lors de l'étape d'échange chlore/fluor.

Le document WO 2009/123328 décrit la fabrication de composés sulfonylimides, avec une réaction entre l'acide amidosulfurique et le chlorure de thionyle, puis avec l'acide chlorosulfonique, pour former le bis(chlorosulfonyl)imide, qui est ensuite soumis à une étape de fluoration. La fluoration est effectuée avec un composé fluoré tel que CuF₂, ZnF₂, SnF₂, PbF₂ ou BiF₃. Ces composés fluorés sont toutefois d'un coût élevé, rendant difficile l'exploitation du procédé à l'échelle industrielle.

Beran et al. (A new Method of the preparation of imido-bis (sulfuric acid) dihalogenide, and the potassium salt of imido-bis(sulfuric acid) difluoride), Zeitschrift Fuer Anorganische Und Allgemeine Chemie, vol. 634, n°1, 2005, p. 55-59) décrit la preparation du F-SO2-NH-SO2-NH comprenant la reaction de l'acide sulfamique avec du chlorure de thionyle et de l'acide chlorosulfonique.

WO2012/160280 concerne la réaction de fluoration du sel Cl-(SO₂)-N⁻-(SO₂)Cl,C⁺ par HF.

Il existe donc encore un besoin de produire des imides contenant un groupement sulfonyle (tels que le LiFSI), notamment selon un procédé pouvant être mis en œuvre à l'échelle industrielle.

### RESUME DE L'INVENTION

L'invention concerne en premier lieu un procédé de préparation d'un composé fluoré de formule :

(III) R₂-(SO₂)-NX-(SO₂)-F

comprenant :
(a) une première étape permettant d'obtenir le composé chloré de formule :

   (II) R₁-(SO₂)-NX-(SO₂)-Cl ;

   cette première étape comprenant la réaction du sulfamide de formule :

   (I) R₀-(SO₂)-NH₂

   avec un acide soufré et un agent chlorant ; et
(b) une deuxième étape permettant d'obtenir le composé fluoré de formule (III), cette deuxième étape comprenant la réaction du composé chloré de formule (II) avec de l'acide fluorhydrique anhydre dans au moins un solvant organique ;
dans lequel :
- X représente soit un atome d'hydrogène soit un cation monovalent M;
- R₁ représente Cl, F, ou un groupement alkyle ou alkoxyalkyle comprenant de 1 à 9 atomes de carbone, et substitué en tout ou partie par du fluor;
- si R₁ représente Cl, alors R₀ représente OH; sinon, R₀ est identique à R₁ ; et
- si R₁ représente Cl, alors R₂ représente F ; sinon, R₂ est identique à R₁.

Selon un mode de réalisation, R₁ est choisi parmi Cl, F, CF₃, CHF₂, CH₂F, C₂HF₄, C₂H₂F₃, C₂H₃F₂, C₂F₅, C₃F₇, C₃H₂F₅, C₃H₄F₃, C₃HF₆, C₄F₉, C₄H₂F₇, C₄H₄F₅, C₅F₁₁, C₃F₆OCF₃, C₂F₄OCF₃, C₂H₂F₂OCF₃, CF₂OCF₃, C₆F₁₃, C₇F₁₅, C₈F₁₇ ou C₉F₁₉.

Selon un mode de réalisation, M représente un cation de métal alcalin, de métal alcalinoterreux ou un cation ammonium quaternaire, et de préférence M représente un cation lithium ou sodium, et de manière plus particulièrement préférée un cation lithium.

Selon un mode de réalisation, l'acide soufré utilisé à la première étape est choisi parmi l'acide chlorosulfonique, l'acide sulfurique, l'oléum et les mélanges de ceux-ci.

Selon un mode de réalisation, l'agent chlorant utilisé à la première étape est choisi parmi le chlorure de thionyle, le chlorure d'oxalyle, le pentachlorure de phosphore, le trichlorure de phosphonyle, le trichlorure de phosphoryle et les mélanges de ceux-ci.

Selon un mode de réalisation :
- un catalyseur est utilisé pour la réaction du sulfamide avec l'acide soufré et l'agent chlorant à la première étape, qui est de préférence choisi parmi une amine tertiaire telle que la méthylamine, la triéthylamine ou la diéthylméthylamine, ou la pyridine et ses dérivés tels que la 2,6-lutidine ; et/ou
- la réaction du sulfamide avec l'acide soufré et l'agent chlorant à la première étape est mise en œuvre à une température comprise entre 30 et 150°C ; et/ou
- la réaction du sulfamide avec l'acide soufré et l'agent chlorant à la première étape est mise en œuvre à une pression comprise entre 1 et 7 bars absolus.

Selon un mode de réalisation :
- le rapport molaire entre l'acide soufré et le sulfamide mis en jeu dans la première étape est compris entre 1 et 5 ; et/ou
- le rapport molaire entre l'agent chlorant et le sulfamide mis en jeu dans la première étape est compris entre 1 et 10.

Selon un mode de réalisation, le solvant organique à la deuxième étape possède un nombre donneur compris entre 1 et 70, et avantageusement entre 5 et 65.

Selon un mode de réalisation, le solvant organique à la deuxième étape est choisi parmi les esters, les nitriles, les dinitriles, les éthers, les diéthers, les amines et les phosphines, et les mélanges de ceux-ci.

Selon un mode de réalisation :
- la réaction du composé chloré de formule (II) avec l'acide fluorhydrique anhydre de la deuxième étape est mise en œuvre à une température comprise entre 0°C et la température d'ébullition du solvant organique, de préférence comprise entre 5°C et la température d'ébullition du solvant organique ; et/ou
- la réaction du composé chloré de formule (II) avec l'acide fluorhydrique anhydre de la deuxième étape est mise en œuvre à une pression comprise entre 0 et 16 bars absolus.

Selon un mode de réalisation, le composé chloré de formule (II) est dissout dans le solvant organique préalablement à la deuxième étape de réaction.

Selon un mode de réalisation :
- le rapport massique entre le composé chloré de formule (II) et le solvant organique mis en jeu dans la réaction du composé chloré de formule (II) avec l'acide fluorhydrique anhydre de la deuxième étape est compris entre 0,001 et 10 et de préférence entre 0,005 et 5 ; et/ou
- le rapport molaire entre le composé chloré de formule (II) et l'acide fluorhydrique mis en jeu dans la réaction du composé chloré de formule (II) avec l'acide fluorhydrique anhydre de la deuxième étape est compris entre 0,01 et 0,5 et de préférence entre 0,05 et 0,5.

Selon un mode de réalisation, la réaction du sulfamide avec l'acide soufré et l'agent chlorant de la première étape fournit le composé chloré de formule :

(IIa) R₁-(SO₂)-NH-(SO₂)-Cl ;

la première étape comprenant en outre la réaction du composé chloré de formule (IIa) avec une base, permettant d'obtenir le composé chloré de formule :

(IIb) R₁-(SO₂)-NM-(SO₂)-Cl ;

dans laquelle M représente un cation monovalent.

Selon un mode de réalisation, ladite base est choisie parmi les carbonates de métal alcalin, les carbonates de métal alcalinoterreux, les hydroxydes de métal alcalin, les hydroxydes de métal alcalinoterreux, les amines tertiaires dans un solvant organique polaire, et les mélanges de ceux-ci.

Selon un mode de réalisation, le procédé comprend, après la deuxième étape :
(c) une troisième étape de neutralisation du composé de formule (III), de préférence par ajout d'une base choisie parmi les carbonates de métal alcalin, les carbonates de métal alcalinoterreux, les hydroxydes de métal alcalin, les hydroxydes de métal alcalinoterreux et les mélanges de ceux-ci.

Selon un mode de réalisation, le composé fluoré de formule (III) obtenu à la deuxième étape est un composé de formule :

(IIIa) R₂-(SO₂)-NH-(SO₂)-F

et la troisième étape de neutralisation permet de convertir le composé de formule (IIIa) en un composé de formule :

(IIIb) R₂-(SO₂)-NM-(SO₂)-F

où M représente un cation monovalent.

Selon un mode de réalisation, le procédé comprend après la deuxième étape ou le cas échéant la troisième étape, une étape finale d'échange de cations, de préférence par mise en contact avec un fluorure, chlorure, carbonate, hydroxyde, sulfate, chlorate, perchlorate, nitrite ou nitrate de métal alcalin ou alcalinoterreux ou d'ammonium quaternaire.

Selon un mode de réalisation, le procédé permet d'obtenir du LiN(FSO₂)₂, du LiN(SO₂CF₃)(SO₂F), du LiN(SO₂C₂F₅)(SO₂F), du LiN(SO₂CF₂OCF₃)(SO₂F), du LiN(SO₂C₃HF₆)(SO₂F), du LiN(SO₂C₄F₉)(SO₂F), du LiN(SO₂C₅F₁₁)(SO₂F), du LiN(SO₂C₆F₁₃)(SO₂F), du LiN(SO₂C₇F₁₅)(SO₂F), du LiN(SO₂C₈F₁₇)(SO₂F) ou du LiN(SO₂C₉F₁₉)(SO₂F), et de préférence du LiN(FSO₂)₂.

L'invention concerne également un procédé de fabrication d'un électrolyte, comprenant la préparation d'un sel d'imide de formule (IIIb) R₂-(SO₂)-NM-(SO₂)-F, où M représente un cation monovalent et R₂ représente un groupement électroattracteur présentant un paramètre σₚ de Hammett positif, par le procédé décrit ci-dessus, et la dissolution de celui-ci dans un solvant, ledit sel d'imide étant de préférence un sel de lithium ou de sodium.

L'invention concerne également un procédé de fabrication d'une batterie ou d'une cellule de batterie, comprenant la fabrication d'un électrolyte selon le procédé ci-dessus et l'insertion de cet électrolyte entre une anode et une cathode.

Il est décrit une composition comprenant au moins 99,9 % en masse d'un sel d'imide de formule :

(IIIb) R₂-(SO₂)-NM-(SO₂)-F

dans laquelle M représente un cation monovalent et R₂ représente un groupement électroattracteur présentant un paramètre σ_{ρ} de Hammett positif, la composition comprenant une teneur massique en fluorures totaux de 1 à 500 ppm, et/ou une teneur massique en chlorures totaux de 1 à 200 ppm.

La teneur massique en fluorures totaux peut aller de 1 à 250 ppm et/ou la teneur massique en chlorures totaux peut aller de 1 à 100 ppm.

La composition peut comprendre une teneur massique en nitrates inférieure ou égale à 250 ppm, de préférence inférieure ou égale à 150 ppm ; et/ou une teneur massique en sulfates inférieure ou égale à 250 ppm, de préférence inférieure ou égale à 150 ppm.

Dans la composition :
- M peut représenter Li ou Na ; et/ou
- R₂ peut représenter F, CF₃, CHF₂, CH₂F, C₂HF₄, C₂H₂F₃, C₂H₃F₂, C2F5, C₃F₇, C₃H₂F₅, C₃H₄F₃, C₃HF₆, C₄F₉, C₄H₂F₇, C₄H₄F₅, C₅F₁₁, C₃F₆OCF₃, C₂F₄OCF₃, C₂H₂F₂OCF₃, CF₂OCF₃, C₆F₁₃, C₇F₁₅, C₈F₁₇ ou C₉F₁₉, F étant préféré.

Le sel d'imide peut être du LiN(FSO₂)₂, du LiN(SO₂CF₃)(SO₂F), du LiN(SO₂C₂F₅)(SO₂F), du LiN(SO₂CF₂OCF₃)(SO₂F), du LiN(SO₂C₃HF₆)(SO₂F), du LiN(SO₂C₄F₉)(SO₂F), du LiN(SO₂C₅F₁₁)(SO₂F), du LiN(SO₂C₆F₁₃)(SO₂F), du LiN(SO₂C₇F₁₅)(SO₂F), du LiN(SO₂C₈F₁₇)(SO₂F) ou du LiN(SO₂C₉F₁₉)(SO₂F), et de préférence du LiN(FSO₂)₂.

La présente invention permet de surmonter les inconvénients de l'état de la technique. Elle fournit plus particulièrement un procédé de production d'imides contenant un groupement sulfonyle (tels que le LiFSI) susceptible d'être mis en œuvre à l'échelle industrielle, et sans entraîner un coût excessif.

Cela est accompli principalement grâce à la réaction de fluoration d'un composé bis(sulfonyl)imide chloré avec de l'acide fluorhydrique anhydre dans un solvant organique. Il a été observé de façon surprenante que cette réaction de fluoration fournit un rendement supérieur à 70%.

Ainsi, la demanderesse a renversé le préjugé illustré dans le document WO 2007/068822.

### DESCRIPTION DE MODES DE REALISATION DE L'INVENTION

L'invention est maintenant décrite plus en détail et de façon non limitative dans la description qui suit.

L'invention prévoit de préparer un composé comprenant au moins un groupe fluorosulfonyle selon un schéma général en au moins deux étapes :
(a) Préparation d'un composé comprenant au moins un groupe chlorosulfonyle.
(b) Fluoration du composé de l'étape (a).

En outre, on peut prévoir de manière optionnelle une troisième étape :
(c) Neutralisation du composé de l'étape (b).

En outre, on peut prévoir de manière optionnelle une quatrième étape, soit après la troisième étape soit directement après la deuxième étape :
(d) Echange de cations.

### Etape (a)

Dans l'étape (a), on fait réagir le sulfamide de formule (I) R₀-(SO₂)-NH₂ avec un acide soufré et un agent chlorant, de sorte à obtenir un composé chloré de formule (II) R₁-(SO₂)-NX-(SO₂)-Cl.

X représente soit un atome d'hydrogène, soit un cation monovalent noté M.

Lorsque X représente un atome d'hydrogène, le composé chloré ci-dessus est le composé de formule (IIa) R₁-(SO₂)-NH-(SO₂)-Cl.

Lorsque X représente un cation monovalent, le composé chloré est le sel de formule (IIb) R₁-(SO₂)-NM-(SO₂)-Cl, qui peut également s'écrire R₁-(SO₂)-N⁻-(SO₂)-Cl, M⁺.

A titre de cation monovalent, on peut utiliser un cation de métal alcalin, de métal alcalinoterreux ou un cation ammonium quaternaire. Le sodium et surtout le lithium sont préférés.

Lorsque le composé chloré (II) obtenu à l'issue de la première étape est le composé de formule (IIa), la réaction du sulfamide avec l'acide soufré et l'agent chlorant permet d'obtenir directement le composé chloré.

Lorsque le composé chloré (II) obtenu à l'issue de la première étape est le composé de formule (IIb), on procède en deux temps :
- dans un premier temps, la réaction du sulfamide avec l'acide soufré et l'agent chlorant, qui permet d'obtenir le composé chloré de formule (IIa) ;
- dans un deuxième temps, la conversion du composé chloré de formule (IIa) en le composé chloré de formule (IIb) par réaction avec une base.

R₁ représente Cl, F, ou un groupement alkyle ou alkoxyalkyle comprenant de 1 à 9 atomes de carbone, et substitué en tout ou partie par du fluor. Des exemples de tels groupements sont les groupements : CF₃, CHF₂, CH₂F, C₂HF₄, C₂H₂F₃, C₂H₃F₂, C₂F₅, C₃F₇, C₃H₂F₅, C₃H₄F₃, C₃HF₆, C₄F₉, C₄H₂F₇, C₄H₄F₅, C₅F₁₁, C₃F₆OCF₃, C₂F₄OCF₃, C₂H₂F₂OCF₃, CF₂OCF₃, C₆F₁₃, C₇F₁₅, C₈F₁₇ et C₉F₁₉.

Lorsque R₁ représente Cl, alors le sulfamide de départ est l'acide amidosulfonique de formule (I') OH-(SO₂)-NH₂ (R₀ représente OH).

Lorsque R₁ représente un autre groupement, alors le sulfamide de départ a pour formule (I") R₁-(SO₂)-NH₂ (R₀ est identique à R₁).

L'acide soufré utilisé pour la réaction peut être l'acide chlorosulfonique CISO₃H, ou alternativement l'acide sulfurique ou l'oléum. On peut également utiliser des combinaisons de ces réactifs.

L'agent chlorant utilisé pour la réaction peut être choisi parmi le chlorure de thionyle SOCl₂, le chlorure d'oxalyle (COCl)₂, le pentachlorure de phosphore PCl₅, le trichlorure de phosphonyle PCl₃ ou le trichlorure de phosphoryle POCl₃. On peut également utiliser des combinaisons de ces réactifs.

On peut également utiliser un catalyseur pour accélérer la réaction, par exemple choisi parmi une amine tertiaire telle que la méthylamine, triéthylamine ou la diéthylméthylamine. On peut également utiliser la pyridine ou un dérivé de celle-ci tel que la 2,6-lutidine. Le rapport molaire entre l'acide soufré et le sulfamide est avantageusement compris entre 1 et 5. Le rapport molaire entre l'agent chlorant et le sulfamide est avantageusement compris entre 1 et 10, et plus particulièrement : entre 1 et 5 lorsque l'acide soufré est l'acide chlorosulfonique ; et entre 2 et 10 lorsque l'acide soufré est l'acide sulfurique ou l'oléum.

La température de réaction est avantageusement comprise entre 30 et 150°C. Le temps de réaction est avantageusement compris entre 1 heure et 7 jours. La réaction peut avantageusement être réalisée sous une pression comprise entre 1 bar absolu et 7 bars absolus.

La réaction conduit à un dégagement gazeux de HCl, ainsi que d'autres gaz qui peuvent être par exemple, selon l'agent chlorant utilisé, du CO, du CO₂ ou du SO₂.

Les éventuels réactifs non réagis ou produits dégradés en solution peuvent être éliminés par une étape de purification par filtration ou par recristallisation dans un solvant apolaire tel que le pentane, le toluène ou le cyclohexane.

En ce qui concerne la réaction optionnelle de conversion du composé chloré de formule (IIa) en composé chloré (sel) de formule (IIb), on procède en faisant réagir le composé chloré de formule (IIa) avec une base qui peut être par exemple un carbonate de métal alcalin ou alcalino-terreux, un hydroxyde de métal alcalin ou alcalino-terreux ou un mélange de ceux-ci, ou encore au moins une amine tertiaire dans un solvant organique de type polaire.

A titre de solvant organique de type polaire on peut notamment utiliser l'acétonitrile, le dioxane, le tétrahydrofurane, l'acétate d'éthyle, l'acétate de butyle et les combinaisons de ceux-ci.

Cette réaction peut être mise en œuvre en dissolvant le composé chloré de formule (IIa) dans le solvant organique, par exemple à une concentration de 10⁻³ à 10 mol/L. La base peut être ajoutée sous forme liquide ou solide. Le rapport molaire base / composé chloré de formule (IIa) peut être par exemple de 1 lorsque la base est un hydroxyde ou une amine, ou de 2 lorsque la base est un carbonate. La température de la réaction peut être par exemple comprise entre -10 et 40°C.

A la fin de la réaction, l'excès de base peut être filtré, et la solution peut être évaporée.

### Etape (b)

Dans l'étape (b), on procède à la fluoration du composé chloré de formule (IIa) ou de formule (IIb) obtenu à l'issue de l'étape (a), de sorte à obtenir le composé fluoré de formule (III) R₁-(SO₂)-NX-(SO₂)-F.

Lorsque la fluoration met en jeu un composé chloré de formule (IIa), le composé fluoré obtenu est le composé de formule (IIIa) R₂-(SO₂)-NH-(SO₂)-F.

Lorsque la fluoration met en jeu un composé chloré de formule (IIb), le composé fluoré obtenu est le sel de formule (IIIb) R₂-(SO₂)-NM-(SO₂)-F, qui peut également s'écrire R₂-(SO₂)-N⁻-(SO₂)-F, M⁺.

Lorsque R₁ représente Cl, R₂ représente F.

Dans tous les autres cas, R₂ est identique à R₁ tel que défini ci-dessus.

De préférence, R₂ représente F, CF₃, CHF₂, CH₂F ou CF₂OCF₃. Il est particulièrement préféré que R₂ représente F.

La réaction de fluoration utilise de l'acide fluorhydrique (HF) anhydre dans un solvant organique.

Le solvant organique possède de préférence un nombre donneur compris entre 1 et 70 et avantageusement compris entre 5 et 65. L'indice donneur d'un solvant représente la valeur -ΔH, ΔH étant l'enthalpie de l'interaction entre le solvant et le pentachlorure d'antimoine (selon la méthode décrite dans Journal of Solution Chemistry, vol. 13, n°9, 1984). Comme solvant organique, on peut citer notamment les esters, les nitriles ou dinitriles, les éthers ou diéthers, les amines ou les phosphines. On peut également utiliser des combinaisons de ceux-ci à titre de solvant organique.

L'acétate de méthyle, l'acétate d'éthyle, l'acétate de butyle, l'acétonitrile, le propionitrile, l'isobutyronitrile, le glutaronitrile, le dioxane, le tétrahydrofurane, la triéthylamine, la tripropylamine, la diéthylisopropylamine, la pyridine, la triméthylphosphine, la triéthylphosphine, la diéthylisopropylphosphine et les mélanges de ceux-ci peuvent notamment convenir comme solvants organiques.

La réaction avec l'HF anhydre peut être mise en œuvre à une température de préférence comprise entre 0°C, de préférence 20°C et la température d'ébullition du solvant organique utilisé. Avantageusement, cette température est comprise entre 5°C, de préférence 25°C et la température d'ébullition du solvant organique.

Selon la présente invention, l'étape de réaction avec l'HF anhydre est mise en œuvre à une pression qui est de préférence comprise entre 0 et 16 bars absolus.

Le composé chloré de formule (II) est de préférence dissout dans le solvant organique préalablement à l'étape de réaction avec l'HF anhydre.

Le rapport massique entre le composé chloré de formule (II) et le solvant organique est de préférence compris entre 0,001 et 10, et avantageusement compris entre 0,005 et 5.

L'HF est introduit dans le milieu réactionnel de préférence sous forme gazeuse.

Le rapport molaire entre le composé chloré de formule (II) et l'HF mis en jeu est de préférence compris entre 0,01 et 0,5 et avantageusement compris entre 0,05 et 0,5.

L'étape de réaction avec l'HF peut être effectuée en milieu fermé ou en milieu ouvert.

Sans vouloir être liée par une théorie, la demanderesse estime que l'utilisation d'un solvant organique donneur permet de former d'un complexe solvant-HF et ainsi d'exalter la nucléophilie de l'atome de fluor. L'utilisation d'un tel complexe permet une fluoration douce du composé chloré de formule (II) en évitant ainsi les réactions parasites de coupure.

Le procédé selon la présente invention permet d'obtenir des rendements de fluoration compris entre 85 et 100%, ce qui représente une nette augmentation en comparaison des procédés de l'art antérieur.

La réaction de fluoration conduit à la formation de HCl, dont la majorité peut être dégazé du milieu réactionnel (tout comme l'HF excédentaire), par exemple par barbotage d'un gaz neutre (tel que l'azote, l'hélium ou l'argon).

Toutefois, de l'HF et/ou de l'HCl résiduels peuvent être dissous dans le milieu réactionnel. Dans le cas de l'HCl les quantités sont très faibles car aux pression et température de travail l'HCl est principalement sous forme gaz.

### Etape (c)

Après l'étape (b), le milieu réactionnel est donc de préférence neutralisé, par exemple à l'aide d'une solution aqueuse de carbonate de métal alcalin ou alcalino-terreux M'CO₃, nH₂O ou d'hydroxyde de métal alcalin ou alcalinoterreux M'OH, nH₂O pour obtenir de préférence un pH supérieur à 4. On peut également utiliser des mélanges de carbonates et/ou d'hydroxydes ci-dessus.

Dans ce qui précède, M' désigne un cation monovalent de métal alcalin ou alcalinoterreux.

L'HF résiduel et/ou l'HCl résiduel dissous dans le solvant réagit avec le carbonate ou hydroxyde ci-dessus, de sorte à former un fluorure de métal alcalin ou alcalino-terreux M'F (ou un mélange de fluorures M'F), respectivement un chlorure de métal alcalin ou alcalino-terreux M'CI (ou un mélange de chlorures M'CI).

La réaction de neutralisation peut être mise en œuvre par exemple par ajout d'une solution aqueuse de la base choisie. Le rapport molaire base / composé fluoré de formule (III) peut être par exemple de 1 à 5 lorsque la base est un hydroxyde, ou de 0,5 à 5 ou de 2 à 10, lorsque la base est un carbonate. La température de la réaction peut être par exemple comprise entre -10 et 40°C.

A la fin de la réaction, l'excès de base peut être filtré, et la solution peut être évaporée. Cela permet également en grande partie l'élimination des fluorures et chlorures formés.

La solution peut ensuite être extraite avec un solvant organique qui peut être par exemple du dichlorométhane, de l'acétonitrile, de l'acétate d'éthyle, de l'acétate de butyle, du diéthylether, du térahydrofurane, du toluène ou un mélange de ceux-ci. Cette extraction peut être réalisée plusieurs fois pour maximiser le rendement de récupération.

La phase organique obtenue peut ensuite être extraite plusieurs fois avec de l'eau pour purifier le produit. La solution organique peut ensuite être évaporée pour fournir le sel d'imide contenant un groupement fluorosulfonyle recherché.

Le sel d'imide ainsi obtenu présente de préférence une teneur massique en fluorures de moins de 500 ppm, et de manière plus particulièrement préférée de moins de 250 ppm.

Et le sel d'imide ainsi obtenu présente de préférence une teneur massique en chlorures de moins de 200 ppm, et de manière plus particulièrement préférée de moins de 100 ppm.

Il faut noter que, dans le cas particulier où l'on obtient à l'issue de la deuxième étape le composé fluoré de formule (IIIa) R₂-(SO₂)-NH-(SO₂)-F, la troisième étape de neutralisation telle que décrite ci-dessus, conduit également à convertir ce composé en le composé fluoré (sel) de formule (IIIb) R₂-(SO₂)-NM-(SO₂)-F, M étant égal à M'.

### Etape (d)

De manière optionnelle, on peut prévoir une étape d'échange de cations à la fin du procédé. Cette étape permet de convertir un composé fluoré de formule (IIIb) R₂-(SO₂)-NM-(SO₂)-F en composé fluoré de formule (IIIc) R₂-(SO₂)-NM"-(SO₂)-F, où M" représente un cation.

M" peut notamment représenter un cation de métal alcalin ou alcalinoterreux ou un cation ammonium quaternaire. Il peut s'agir par exemple du cation lithium ou sodium, et plus particulièrement lithium.

Cette étape d'échange de cations est effectuée en mettant le composé fluoré de formule (IIIb) en présence d'un sel du cation M", qui peut être un sel fluorure, chlorure, carbonate, hydroxyde, sulfate, chlorate, perchlorate, nitrite ou nitrate. On peut également utiliser une combinaison de ces composés.

La réaction peut être réalisée par exemple dans l'eau ou dans un solvant organique polaire tel que notamment l'acétonitrile, la N-méthylpyrrolidone, le diméthylformamide, le diméthylsulfoxyde, le nitrométhane, le dioxane, le tétrahydrofurane, l'acétate d'éthyle, l'acétate de butyle et les mélanges de ceux-ci.

La réaction peut être réalisée par exemple à une température comprise entre 0° et la température d'ébullition du solvant utilisé.

Le temps de réaction peut être compris par exemple entre 1 heure et 5 jours.

Le rapport molaire entre le sel du cation M" et le sel d'imide peut être compris par exemple entre 0,9 et 5. La concentration en sel d'imide dans l'eau ou le solvant organique peut être comprise par exemple entre 0.001 et 5 mol/L.

Dans le cas particulier où le solvant utilisé est l'eau, le milieu réactionnel peut ensuite être extrait avec un solvant organique qui peut être notamment le dichlorométhane, l'acétonitrile, l'acétate d'éthyle, l'acétate de butyle, le diéthylether, le tétrahydrofurane, le toluène ou les mélanges de ceux-ci. Cette extraction peut être réalisée plusieurs fois pour maximiser le rendement de récupération. La phase organique est ensuite évaporée pour obtenir le sel d'imide de formule (IIIc).

Le procédé selon la présente invention est particulièrement intéressant pour fabriquer les sels d'imide suivants : LiN(SO₂F)₂, LiNSO₂CF₃SO₂F, LiNSO₂C₂F₅SO₂F, LiNSO₂CF₂OCF₃SO₂F, LiNSO₂C₃HF₆SO₂F, LiNSO₂C₄F₉SO₂F, LiNSO₂C₅F₁₁SO₂F, LiNSO₂C₆F₁₃SO₂F, LiNSO₂C₇F₁₅SO₂F, LiNSO₂C₈F₁₇SO₂F et LiNSO₂C₉F₁₉SO₂F.

De préférence, ces sels sont obtenus avec une pureté au moins égale à 99,5 % en poids, avantageusement au moins égale à 99,9 % en poids.

Les impuretés, telles que LiCl, LiF ou NaCl, NaF et FSO₃Na, éventuellement présentes dans le sel d'imide, représentent chacune, de préférence, moins de 1000 ppm, avantageusement moins de 500 ppm.

L'impureté FSO₃Li est éventuellement présente à une concentration inférieure à 50 ppm, de préférence inférieure à 5 ppm.

Les nitrates et sulfates, éventuellement présents dans le sel d'imide, sont avantageusement respectivement présents à une concentration massique de moins de 250 ppm, et de préférence de moins de 150 ppm.

Comme indiqué précédemment, la teneur en fluorures éventuellement présents est de préférence de moins de 500 ppm, et de manière plus particulièrement préférée de moins de 250 ppm.

Comme indiqué précédemment, la teneur en chlorures éventuellement présents est de préférence de moins de 200 ppm, et de manière plus particulièrement préférée de moins de 100 ppm.

Ces concentrations d'impuretés sont des concentrations massiques par rapport à la masse de sel d'imide recherché.

Le sel d'imide obtenu est de préférence essentiellement exempt d'eau et d'impuretés constituées de sels formés d'un cation issu des groupes 11 à 15 et périodes 4 à 6 du tableau périodique (par exemple Zn, Cu, Sn, Pb, Bi).

Ces impuretés sont néfastes aux performances des batteries Li-ion ou Na-ion de par leur activité électrochimique.

### Préparation d'un électrolyte

Le sel d'imide préparé comme décrit ci-dessus peut être utilisé pour la préparation d'un électrolyte, en le dissolvant dans un solvant approprié.

Par exemple, ainsi que cela est décrit dans le document J. Electrochemical Society, 2011, 158, A74-82, le LiFSI peut être dissous à une concentration de 1 mol/L dans un mélange d'éthylenecarbonate (EC), de diméthylcarbonate (DMC) et d'éthylméthylcarbonate (EMC) à 5 pour 2 pour 3 en volume ; un tel électrolyte présente montre une très bonne conductivité, une bonne stabilité en cyclage et une corrosion de l'aluminium au-dessus de 4,2 V.

Cet électrolyte peut ensuite être utilisé pour la fabrication de batteries ou de cellules de batterie, en le disposant entre une cathode et une anode, de manière connue en soi.

### EXEMPLES

Les exemples suivants illustrent l'invention sans la limiter.

### Exemple 1

De l'acide sulfamique (1 éq, 0,515 mol, 50 g) est introduit dans un ballon avec du chlorure de thionyle (3,75 éq, 1,93 mol, 229,8 g) et on coule de l'acide sulfurique à 95 % (1 éq, 0,515 mol, 53,1 g) à température ambiante. On maintient à reflux du chlorure de thionyle pendant 24 heures sous agitation. Le dichloré obtenu au final est d'apparence jaune clair, avec de l'acide sulfamique résiduel non solubilisé. On filtre pour éliminer l'acide sulfamique (23,6 g) puis on évapore sous vide le chlorure de thionyle.

### Exemple 2

Dans un ballon ou un réacteur en verre, on introduit 1 éq d'acide sulfamique (0,25 mol, 24,25 g), puis du chlorure de thionyle (2,75 éq, 0,69 mol, 81,9 g). Ensuite, sous agitation, on coule à température ambiante, très lentement, l'acide chlorosulfonique (2 éq, 0,5 mol, 58,25 g). On amène à reflux progressivement du chlorure de thionyle (bain d'huile à 90 °C) et on laisse ainsi pendant 24 heures, toujours sous agitation. On observe un dégagement gazeux qui est piégé dans l'eau en sortie de réacteur. Au final, le produit récupéré dans le ballon est liquide, légèrement orangé, très fumant.

### Exemple 3

Dans un autoclave de 800 mL, 28 g de (CISO₂)₂NH sont dissous dans 50 mL d'acétonitrile. 10 g d'HF sont ensuite additionnés. La pression est alors de 0,34 bar absolu et la température est maintenue à 10°C. La réaction est laissée sous agitation en milieu fermé pendant 18 heures. L'HF, en excès, est éliminé par un balayage d'un gaz inerte. Le milieu réactionnel est ensuite traité avec du carbonate de lithium. La solution est filtrée puis évaporée et le résidu est analysé par RMN ¹⁹F. L'analyse montre la présence de 85 % de produit totalement fluoré (FSO₂)₂NLi, 7,5% de FSO₃Li et 7,5% de FSO₂NH₂. Ces deux derniers sont les composés formés lors de la dégradation du produit de départ.

### Exemple 4

Dans un autoclave de 800 mL, 31,7 g de (CISO2)₂NH sont dissous dans 50 mL d'acétonitrile. 10 g d'HF sont ensuite additionnés. La pression est alors de 0,75 bar absolu et la température est maintenue à 20°C. La réaction est laissée sous agitation en milieu fermé pendant 18 heures. L'HF, en excès, est éliminé par pompage. Le milieu réactionnel est ensuite traité avec du carbonate de lithium. La solution est filtrée puis évaporé et le résidu est analysé par RMN ¹⁹F. L'analyse montre la présence de 100 % de produit totalement fluoré (FSO₂)₂NLi et l'absence des produits de dégradation FSO₃Li et FSO₂NH₂.

### Exemple 5

Dans un autoclave de 800 mL, 61 g de (CISO₂)₂NH sont dissous dans 50 mL de 1,4-dioxane. 20 g d'HF sont ensuite additionnés. La pression est alors de 2,3 bars absolus et la température est maintenue à 25°C. La réaction est laissée sous agitation en milieu fermé pendant 18 heures. L'HF, en excès, est éliminé par pompage. Le milieu réactionnel est ensuite traité avec du carbonate de lithium. La solution est filtrée puis évaporé et le résidu est analysé par RMN ¹⁹F. L'analyse montre la présence de 100 % de produit totalement fluoré (FSO₂)₂NLi et l'absence des produits de dégradation FSO₃Li et FSO₂NH₂.

### Exemple 6

Dans un autoclave de 800 mL, 65 g de (CISO₂)₂NH sont dissous dans 50 mL de 1,4-dioxane. 20 g d'HF sont ensuite additionnés. La pression est de 0 bars absolus et la température est maintenue à 25°C. La réaction est laissée sous agitation en milieu ouvert pendant 3 heures. L'HF, en excès, est éliminé par balayage d'un gaz inerte. Le milieu réactionnel est ensuite traité avec du carbonate de lithium. La solution est filtrée puis évaporé et le résidu est analysé par RMN ¹⁹F. L'analyse montre la présence de 100 % de produit totalement fluoré (FSO₂)₂NLi et l'absence des produits de dégradation FSO₃Li et FSO₂NH₂.

## Revendications

1. Procédé de préparation d'un composé fluoré de formule :
(III) R₂-(SO₂)-NX-(SO₂)-F
comprenant :
(a) une première étape permettant d'obtenir le composé chloré de formule :
(II) R₁-(SO₂)-NX-(SO₂)-Cl ;
cette première étape comprenant la réaction du sulfamide de formule :
(I) R₀-(SO₂)-NH₂
avec un acide soufré et un agent chlorant ; et
(b) une deuxième étape permettant d'obtenir le composé fluoré de formule (III), cette deuxième étape comprenant la réaction du composé chloré de formule (II) avec de l'acide fluorhydrique anhydre dans au moins un solvant organique ;
dans lequel :
- X représente soit un atome d'hydrogène soit un cation monovalent M ;
- R₁ représente Cl, F ou un groupement alkyle ou alkoxyalkyle comprenant de 1 à 9 atomes de carbone, substitué en tout ou partie par du fluor ;
- si R₁ représente Cl, alors R₀ représente OH ; sinon, R₀ est identique à R₁ ; et
- si R₁ représente Cl, alors R₂ représente F; sinon, R₂ est identique à R₁.

2. Procédé selon la revendication 1, dans lequel R₁ est choisi parmi Cl, F, CF₃, CHF₂, CH₂F, C₂HF₄, C₂H₂F₃, C₂H₃F₂, C₂F₅, C₃F₇, C₃H₂F₅, C₃H₄F₃, C₃HF₆, C₄F₉, C₄H₂F₇, C₄H₄F₅, C₅F₁₁, C₃F₆OCF₃, C₂F₄OCF₃, C₂H₂F₂OCF₃, CF₂OCF₃, C₆F₁₃, C₇F₁₅, C₈F₁₇ ou C₉F₁₉.

3. Procédé selon la revendication 1 ou 2, dans lequel M représente un cation de métal alcalin, de métal alcalinoterreux ou un cation ammonium quaternaire, et dans lequel de préférence M représente un cation lithium ou sodium, et de manière plus particulièrement préférée un cation lithium.

4. Procédé selon l'une des revendications 1 à 3, dans lequel l'acide soufré utilisé à la première étape est choisi parmi l'acide chlorosulfonique, l'acide sulfurique, l'oléum et les mélanges de ceux-ci.

5. Procédé selon l'une des revendications 1 à 4, dans lequel l'agent chlorant utilisé à la première étape est choisi parmi le chlorure de thionyle, le chlorure d'oxalyle, le pentachlorure de phosphore, le trichlorure de phosphonyle, le trichlorure de phosphoryle et les mélanges de ceux-ci.

6. Procédé selon l'une des revendications 1 à 5, dans lequel :
- un catalyseur est utilisé pour la réaction du sulfamide avec l'acide soufré et l'agent chlorant à la première étape, qui est de préférence choisi parmi une amine tertiaire telle que la méthylamine, la triéthylamine ou la diéthylméthylamine, ou la pyridine et ses dérivés tels que la 2,6-lutidine ; et/ou
- la réaction du sulfamide avec l'acide soufré et l'agent chlorant à la première étape est mise en œuvre à une température comprise entre 30 et 150°C ; et/ou
- la réaction du sulfamide avec l'acide soufré et l'agent chlorant à la première étape est mise en œuvre à une pression comprise entre 1 et 7 bars absolus.

7. Procédé selon l'une des revendications 1 à 6, dans lequel :
- le rapport molaire entre l'acide soufré et le sulfamide mis en jeu dans la première étape est compris entre 1 et 5 ; et/ou
- le rapport molaire entre l'agent chlorant et le sulfamide mis en jeu dans la première étape est compris entre 1 et 10.

8. Procédé selon l'une des revendications 1 à 7, dans lequel le solvant organique à la deuxième étape possède un nombre donneur compris entre 1 et 70, et avantageusement entre 5 et 65.

9. Procédé selon l'une des revendications 1 à 8, dans lequel le solvant organique à la deuxième étape est choisi parmi les esters, les nitriles, les dinitriles, les éthers, les diéthers, les amines et les phosphines, et les mélanges de ceux-ci.

10. Procédé selon l'une des revendications 1 à 9, dans lequel :
- la réaction du composé chloré de formule (II) avec l'acide fluorhydrique anhydre de la deuxième étape est mise en œuvre à une température comprise entre 0°C et la température d'ébullition du solvant organique, de préférence comprise entre 5°C et la température d'ébullition du solvant organique ; et/ou
- la réaction du composé chloré de formule (II) avec l'acide fluorhydrique anhydre de la deuxième étape est mise en œuvre à une pression comprise entre 0 et 16 bars absolus.

11. Procédé selon l'une des revendications 1 à 10, dans lequel le composé chloré de formule (II) est dissout dans le solvant organique préalablement à la deuxième étape de réaction.

12. Procédé selon l'une des revendications 1 à 11, dans lequel :
- le rapport massique entre le composé chloré de formule (II) et le solvant organique mis en jeu dans la réaction du composé chloré de formule (II) avec l'acide fluorhydrique anhydre de la deuxième étape est compris entre 0,001 et 10 et de préférence entre 0,005 et 5 ; et/ou
- le rapport molaire entre le composé chloré de formule (II) et l'acide fluorhydrique mis en jeu dans la réaction du composé chloré de formule (II) avec l'acide fluorhydrique anhydre de la deuxième étape est compris entre 0,01 et 0,5 et de préférence entre 0,05 et 0,5.

13. Procédé selon l'une des revendications 1 à 12, dans lequel la réaction du sulfamide avec l'acide soufré et l'agent chlorant de la première étape fournit le composé chloré de formule :
(IIa) R₁-(SO₂)-NH-(SO₂)-Cl ;
la première étape comprenant en outre la réaction du composé chloré de formule (IIa) avec une base, permettant d'obtenir le composé chloré de formule :
(IIb) R₁-(SO₂)-NM-(SO₂)-Cl
dans laquelle M représente un cation monovalent.

14. Procédé selon la revendication 13, dans lequel ladite base est choisie parmi les carbonates de métal alcalin, les carbonates de métal alcalinoterreux, les hydroxydes de métal alcalin, les hydroxydes de métal alcalinoterreux, les amines tertiaires dans un solvant organique polaire, et les mélanges de ceux-ci.

15. Procédé selon l'une des revendications 1 à 14, comprenant, après la deuxième étape :
(c) une troisième étape de neutralisation du composé de formule (III), de préférence par ajout d'une base choisie parmi les carbonates de métal alcalin, les carbonates de métal alcalinoterreux, les hydroxydes de métal alcalin, les hydroxydes de métal alcalinoterreux et les mélanges de ceux-ci.

16. Procédé selon la revendication 15, dans lequel le composé fluoré de formule (III) obtenu à la deuxième étape est un composé de formule :
(IIIa) R₂-(SO₂)-NH-(SO₂)-F
et dans lequel la troisième étape de neutralisation permet de convertir le composé de formule (IIIa) en un composé de formule :
(IIIb) R₂-(SO₂)-NM-(SO₂)-F
où M représente un cation monovalent.

17. Procédé selon l'une des revendications 1 à 16, comprenant, après la deuxième étape ou le cas échéant la troisième étape, une étape finale d'échange de cations, de préférence par mise en contact avec un fluorure, chlorure, carbonate, hydroxyde, sulfate, chlorate, perchlorate, nitrite ou nitrate de métal alcalin ou alcalinoterreux ou d'ammonium quaternaire.

18. Procédé selon l'une des revendications 1 à 17, permettant d'obtenir du LiN(SO₂F)₂, du LiN(SO₂CF₃)(SO₂F), du LiN(SO₂C₂F₅)(SO₂F), du LiN(SO₂CF₂OCF₃)(SO₂F), du LiN(SO₂C₃HF₆)(SO₂F), du LiN(SO₂C₄F₉)(SO₂F), du LiN(SO₂C₅F₁₁)(SO₂F), du LiN(SO₂C₆F₁₃)(SO₂F), du LiN(SO₂C₇F₁₅)(SO₂F), du LiN(SO₂C₈F₁₇)(SO₂F) ou du LiN(SO₂C₉F₁₉)(SO₂F), et de préférence du LiN(SO₂F)₂.

19. Procédé de fabrication d'un électrolyte, comprenant la préparation d'un sel d'imide de formule (IIIb) R₂-(SO₂)-NM-(SO₂)-F, où M représente un cation monovalent et R₂ représente un groupement électroattracteur présentant un paramètre σₚ de Hammett positif, par le procédé de l'une des revendications 1 à 18, et la dissolution de celui-ci dans un solvant, ledit sel d'imide étant de préférence un sel de lithium, de sodium ou de potassium.

20. Procédé de fabrication d'une batterie ou d'une cellule de batterie, comprenant la fabrication d'un électrolyte selon la revendication 19 et l'insertion de cet électrolyte entre une anode et une cathode.

## Patentansprüche

1. Verfahren zur Herstellung einer Fluorverbindung der Formel:
(III) R₂-(SO₂)-NX-(SO₂)-F,
umfassend:
(a) einen ersten Schritt zum Erhalt der Chlorverbindung der Formel:
(II) R₁- (SO₂) -NX- (SO₂) -Cl;
wobei dieser erste Schritt die Umsetzung des Sulfamids der Formel:
(I) R₀-(SO₂)-NH₂
mit einer schwefelhaltigen Säure und einem Chlorierungsmittel umfasst; und
(b) einen zweiten Schritt zum Erhalt der Fluorverbindung der Formel (III), wobei dieser zweite Schritt die Umsetzung der Chlorverbindung der Formel (II) mit wasserfreier Fluorwasserstoffsäure in mindestens einem organischen Lösungsmittel umfasst;
wobei:
- X für ein Wasserstoffatom oder für ein einwertiges Kation M steht;
- R₁ für Cl, F oder eine Alkyl- oder Alkoxyalkylgruppe mit 1 bis 9 Kohlenstoffatomen, die ganz oder teilweise durch Fluor substituiert ist, steht;
- dann, wenn R₁ für Cl steht, R₀ für OH steht; ansonsten R₀ mit R₁ identisch ist; und
- dann, wenn R₁ für Cl steht, R₂ für F steht; ansonsten R₂ mit R₁ identisch ist.

2. Verfahren nach Anspruch 1, wobei R₁ aus Cl, F, CF₃, CHF₂, CH₂F, C₂HF₄, C₂H₂F₃, C₂H₃F₂, C₂F₅, C₃F₇, C₃H₂F₅, C₃H₄F₃, C₃HF₆, C₄F₉, C₄H₂F₇, C₄H₄F₅, C₅F₁₁, C₃F₆OCF₃, C₂F₄OCF₃, C₂H₂F₂OCF₃, CF₂OCF₃, C₆F₁₃, C₇F₁₅, C₈F₁₇ oder C₉F₁₉ ausgewählt ist.

3. Verfahren nach Anspruch 1 oder 2, wobei M für ein Alkalimetall- oder Erdalkalimetallkation oder ein quaternäres Ammoniumkation steht und wobei vorzugsweise M für ein Lithium- oder Natriumkation und weiter bevorzugt für ein Lithiumkation steht.

4. Verfahren nach einem der Ansprüche 1 bis 3, wobei die im ersten Schritt verwendete schwefelhaltige Säure aus Chlorsulfonsäure, Schwefelsäure, Oleum und Mischungen davon ausgewählt wird.

5. Verfahren nach einem der Ansprüche 1 bis 4, wobei das im ersten Schritt verwendete Chlorierungsmittel aus Thionylchlorid, Oxalylchlorid, Phosphorpentachlorid, Phosphonyltrichlorid, Phosphoryltrichlorid und Mischungen davon ausgewählt wird.

6. Verfahren nach einem der Ansprüche 1 bis 5, wobei:
- für die Umsetzung des Sulfamids mit der schwefelhaltigen Säure und dem Chlorierungsmittel im ersten Schritt ein Katalysator verwendet wird, der vorzugsweise aus einem tertiären Amin wie Methylamin, Triethylamin oder Diethylmethylamin oder Pyridin und Derivaten davon wie 2,6-Lutidin ausgewählt wird; und/oder
- die Umsetzung des Sulfamids mit der schwefelhaltigen Säure und dem Chlorierungsmittel im ersten Schritt bei einer Temperatur zwischen 30 und 150 °C durchgeführt wird; und/oder
- die Umsetzung des Sulfamids mit der schwefelhaltigen Säure und dem Chlorierungsmittel im ersten Schritt bei einem Druck zwischen 1 und 7 bar absolut durchgeführt wird.

7. Verfahren nach einem der Ansprüche 1 bis 6, wobei:
- das Molverhältnis zwischen der schwefelhaltigen Säure und dem Sulfamid, das im ersten Schritt verwendet wird, zwischen 1 und 5 liegt; und/oder
- das Molverhältnis zwischen dem Chlorierungsmittel und dem Sulfamid, das im ersten Schritt verwendet wird, zwischen 1 und 10 liegt.

8. Verfahren nach einem der Ansprüche 1 bis 7, wobei das organische Lösungsmittel im zweiten Schritt eine Donorzahl zwischen 1 und 70 und vorteilhafterweise zwischen 5 und 65 aufweist.

9. Verfahren nach einem der Ansprüche 1 bis 8, wobei das organische Lösungsmittel im zweiten Schritt aus Estern, Nitrilen, Dinitrilen, Ethern, Diethern, Aminen und Phosphinen und Mischungen davon ausgewählt wird.

10. Verfahren nach einem der Ansprüche 1 bis 9, wobei:
- die Umsetzung der Chlorverbindung der Formel (II) mit wasserfreier Fluorwasserstoffsäure im zweiten Schritt bei einer Temperatur zwischen 0 °C und dem Siedepunkt des organischen Lösungsmittels und vorzugsweise zwischen 5 °C und dem Siedepunkt des organischen Lösungsmittels durchgeführt wird; und/oder
- die Umsetzung der Chlorverbindung der Formel (II) mit wasserfreier Fluorwasserstoffsäure im zweiten Schritt bei einem Druck zwischen 0 und 16 bar absolut durchgeführt wird.

11. Verfahren nach einem der Ansprüche 1 bis 10, wobei die Chlorverbindung der Formel (II) vor dem zweiten Reaktionsschritt in dem organischen Lösungsmittel gelöst wird.

12. Verfahren nach einem der Ansprüche 1 bis 11, wobei:
- das Massenverhältnis zwischen der Chlorverbindung der Formel (II) und dem organischen Lösungsmittel, das bei der Umsetzung der Chlorverbindung der Formel (II) mit wasserfreier Fluorwasserstoffsäure im zweiten Schritt eingesetzt wird, zwischen 0,001 und 10 und vorzugsweise zwischen 0,005 und 5 liegt; und/oder
- das Molverhältnis zwischen der Chlorverbindung der Formel (II) und wasserfreier Fluorwasserstoffsäure, das bei der Umsetzung der Chlorverbindung der Formel (II) mit wasserfreier Fluorwasserstoffsäure im zweiten Schritt eingesetzt wird, zwischen 0,01 und 0,5 und vorzugsweise zwischen 0,05 und 0,5 liegt.

13. Verfahren nach einem der Ansprüche 1 bis 12, wobei die Umsetzung des Sulfamids mit der schwefelhaltigen Säure und dem Chlorierungsmittel im ersten Schritt die Chlorverbindung der Formel:
(IIa) R₁- (SO₂) -NH- (SO₂) -Cl
liefert;
wobei der erste Schritt außerdem die Umsetzung der Chlorverbindung der Formel (IIa) mit einer Base zum Erhalt der Chlorverbindung der Formel:
(IIb) R₁-(SO₂)-NM-(SO₂)-Cl
wobei M für ein einwertiges Kation steht, umfasst.

14. Verfahren nach Anspruch 13, wobei die Base aus Alkalimetallcarbonaten, Erdalkalimetallcarbonaten, Alkalimetallhydroxiden, Erdalkalimetallhydroxiden, tertiären Aminen in einem polaren organischen Lösungsmittel und Mischungen davon ausgewählt wird.

15. Verfahren nach einem der Ansprüche 1 bis 14, die nach dem zweiten Schritt Folgendes umfasst:
(c) einen dritten Schritt der Neutralisation der Verbindung der Formel (III), vorzugsweise durch Zugabe einer Base, die aus Alkalimetallcarbonaten, Erdalkalimetallcarbonaten, Alkalimetallhydroxiden, Erdalkalimetallhydroxiden und Mischungen davon ausgewählt wird.

16. Verfahren nach Anspruch 15, wobei es sich bei der im zweiten Schritt erhaltenen Fluorverbindung der Formel (III) um eine Verbindung der Formel:
(IIIa) R₂-(SO₂)-NH-(SO₂)-F
handelt und wobei der dritte Schritt der Neutralisation die Umwandlung der Verbindung (IIIa) in eine Verbindung der Formel:
(IIIb) R₂-(SO₂)-NM-(SO₂)-F
wobei M für ein einwertiges Kation steht, ermöglicht.

17. Verfahren nach einem der Ansprüche 1 bis 16, das nach dem zweiten Schritt oder gegebenenfalls dem dritten Schritt einen letzten Schritt des Kationenaustauschs umfasst, vorzugsweise durch Inkontaktbringen mit einem Alkalimetall- oder Erdalkalimetall- oder quaternären Ammoniumfluorid, -chlorid, -carbonat, -hydroxid, -sulfat, -chlorat, -perchlorat, -nitrit oder -nitrat.

18. Verfahren nach einem der Ansprüche 1 bis 17 zum Erhalt von LiN (SO₂F)₂, LiN (SO₂CF₃) (SO₂F) , LiN (SO₂C₂F₅) (SO₂F) , LiN (SO₂CF₂OCF₃) (SO₂F), LiN (SO₂C₃HF₆) (SO₂F), LiN (SO₂C₄F₉) (SO₂F), LiN (SO₂C₅F₁₁) (SO₂F) , LiN (SO₂C₆F₁₃) (SO₂F), LiN (SO₂C₇F₁₅) (SO₂F) , LiN (SO₂C₈F₁₇) (SO₂F) oder LiN (SO₂C₉F₁₉) (SO₂F) und vorzugsweise LiN (SO₂F)₂.

19. Verfahren zur Herstellung eines Elektrolyts, umfassend die Herstellung eines Imidsalzes der Formel (IIIb) R₂-(SO₂)-NM-(SO₂)-F, wobei M für ein einwertiges Kation steht und R₂ für eine elektronenanziehende Gruppe mit einem positiven Hammett-Parameter σₚ steht, durch das Verfahren nach einem der Ansprüche 1 bis 18 und das Auflösen des Imidsalzes in einem Lösungsmittel, wobei es sich bei dem Imidsalz vorzugsweise um ein Lithium-, Natrium- oder Kaliumsalz handelt.

20. Verfahren zur Herstellung einer Batterie oder einer Batteriezelle, umfassend die Herstellung eines Elektrolyts nach Anspruch 19 und das Einbringen dieses Elektrolyts zwischen einer Anode und einer Kathode.

## Claims

1. Process for preparing a fluoro compound of formula:
(III) R₂-(SO₂)-NX-(SO₂)-F comprising:
(a) a first step for obtaining the chloro compound of formula:
(II) R₁-(SO₂)-NX-(SO₂)-Cl;
this first step comprising the reaction of the sulfamide of formula:
(I) R₀-(SO₂)-NH₂
with a sulfureous acid and a chlorinating agent; and
(b) a second step for obtaining the fluoro compound of formula (III), this second step comprising the reaction of the chloro compound of formula (II) with anhydrous hydrofluoric acid in at least one organic solvent;
in which:
- X represents either a hydrogen atom or a monovalent cation M;
- R₁ represents Cl, F or an alkyl or alkoxyalkyl group comprising from 1 to 9 carbon atoms, and totally or partially substituted with fluorine;
- if R₁ represents Cl, then R₀ represents OH; otherwise, R₀ is identical to R₁; and
- if R₁ represents Cl, then R₂ represents F; otherwise, R₂ is identical to R₁.

2. Process according to Claim 1, in which R₁ is chosen from Cl, F, CF₃, CHF₂, CH₂F, C₂HF₄, C₂H₂F₃, C₂H₃F₂, C₂F₅, C₃F₇, C₃H₂F₅, C₃H₄F₃, C₃HF₆, C₄F₉, C₄H₂F₇, C₄H₄F₅, C₅F₁₁, C₃F₆OCF₃, C₂F₄OCF₃, C₂H₂F₂OCF₃, CF₂OCF₃, C₆F₁₃, C₇F₁₅, C₈F₁₇ and C₉F₁₉.

3. Process according to Claim 1 or 2, in which M represents an alkali metal or alkaline-earth metal cation or a quaternary ammonium cation, and in which, preferably, M represents a lithium or sodium cation and more particularly preferably a lithium cation.

4. Process according to one of Claims 1 to 3, in which the sulfureous acid used in the first step is chosen from chlorosulfonic acid, sulfuric acid, oleum and mixtures thereof.

5. Process according to one of Claims 1 to 4, in which the chlorinating agent used in the first step is chosen from thionyl chloride, oxalyl chloride, phosphorus pentachloride, phosphonyl trichloride, phosphoryl trichloride and mixtures thereof.

6. Process according to one of Claims 1 to 5, in which:
- a catalyst is used for the reaction of the sulfamide with the sulfureous acid and the chlorinating agent in the first step, which is preferably chosen from a tertiary amine such as methylamine, triethylamine or diethylmethylamine, or pyridine and derivatives thereof such as 2,6-lutidine; and/or
- the reaction of the sulfamide with the sulfureous acid and the chlorinating agent in the first step is performed at a temperature of between 30 and 150°C; and/or
- the reaction of the sulfamide with the sulfureous acid and the chlorinating agent in the first step is performed at a pressure of between 1 and 7 bar absolute.

7. Process according to one of Claims 1 to 6, in which:
- the mole ratio between the sulfureous acid and the sulfamide used in the first step is between 1 and 5; and/or
- the mole ratio between the chlorinating agent and the sulfamide used in the first step is between 1 and 10.

8. Process according to one of Claims 1 to 7, in which the organic solvent in the second step has a donor number of between 1 and 70, and advantageously between 5 and 65.

9. Process according to one of Claims 1 to 8, in which the organic solvent in the second step is chosen from esters, nitriles, dinitriles, ethers, diethers, amines and phosphines, and mixtures thereof.

10. Process according to one of Claims 1 to 9, in which:
- the reaction of the chloro compound of formula (II) with anhydrous hydrofluoric acid of the second step is performed at a temperature of between 0°C and the boiling point of the organic solvent, preferably between 5°C and the boiling point of the organic solvent; and/or
- the reaction of the chloro compound of formula (II) with anhydrous hydrofluoric acid of the second step is performed at a pressure of between 0 and 16 bar absolute.

11. Process according to one of Claims 1 to 10, in which the chloro compound of formula (II) is dissolved in the organic solvent prior to the second reaction step.

12. Process according to one of Claims 1 to 11, in which:
- the mass ratio between the chloro compound of formula (II) and the organic solvent used in the reaction of the chloro compound of formula (II) with anhydrous hydrofluoric acid of the second step is between 0.001 and 10 and preferably between 0.005 and 5; and/or
- the mole ratio between the chloro compound of formula (II) and hydrofluoric acid used in the reaction of the chloro compound of formula (II) with anhydrous hydrofluoric acid of the second step is between 0.01 and 0.5 and preferably between 0.05 and 0.5.

13. Process according to one of Claims 1 to 12, in which the reaction of the sulfamide with the sulfureous acid and the chlorinating agent of the first step provides the chloro compound of formula:
(IIa) R₁-(SO₂)-NH-(SO₂)-Cl;
the first step also comprising the reaction of the chloro compound of formula (IIa) with a base, for obtaining the chloro compound of formula:
(IIb) R₁-(SO₂)-NM-(SO₂)-Cl
in which M represents a monovalent cation.

14. Process according to Claim 13, in which said base is chosen from alkali metal carbonates, alkaline-earth metal carbonates, alkali metal hydroxides, alkaline-earth metal hydroxides, tertiary amines in a polar organic solvent, and mixtures thereof.

15. Process according to one of Claims 1 to 14, comprising, after the second step:
(c) a third step of neutralization of the compound of formula (III), preferably by adding a base chosen from alkali metal carbonates, alkaline-earth metal carbonates, alkali metal hydroxides, alkaline-earth metal hydroxides and mixtures thereof.

16. Process according to Claim 15, in which the fluoro compound of formula (III) obtained in the second step is a compound of formula:
(IIIa) R₂-(SO₂)-NH-(SO₂)-F
and in which the third step of neutralization allows the compound of formula (IIIa) to be converted into a compound of formula:
(IIIb) R₂-(SO₂)-NM-(SO₂)-F
in which M represents a monovalent cation.

17. Process according to one of Claims 1 to 16, comprising, after the second step or, where appropriate, the third step, a final step of cation exchange, preferably by placing in contact with an alkali metal or alkaline-earth metal or quaternary ammonium fluoride, chloride, carbonate, hydroxide, sulfate, chlorate, perchlorate, nitrite or nitrate.

18. Process according to one of Claims 1 to 17, for obtaining LiN (SO₂F)₂, LiN(SO₂CF₃) (SO₂F) , LiN (SO₂C₂F₅) (SO₂F) , LiN (SO₂CF₂OCF₃) (SO₂F) , LiN (SO₂C₃HF₆) (SO₂F) , LiN (SO₂C₄F₉) (SO₂F) , LiN (SO₂C₅F₁₁) (SO₂F) , LiN (SO₂C₆F₁₃) (SO₂F) , LiN (SO₂C₇F₁₅) (SO₂F) , LiN (SO₂C₈F₁₇) (SO₂F) or LiN (SO₂C₉F₁₉) (SO₂F) , and preferably LiN(SO₂F)₂.

19. Process for manufacturing an electrolyte, comprising the preparation of an imide salt of formula (IIIb) R₂-(SO₂)-NM-(SO₂)-F, in which M represents a monovalent cation and R₂ represents an electron-withdrawing group having a positive Hammett parameter σₚ, via the process of one of Claims 1 to 18, and dissolution thereof in a solvent, said imide salt preferably being a lithium, sodium or potassium salt.

20. Process for manufacturing a battery or a battery cell, comprising the manufacture of an electrolyte according to Claim 19 and the insertion of this electrolyte between an anode and a cathode.
